Europäisches Patentamt

European Patent Office

Office européen des brevets

(19)

(11) EP 0 707 844 A2

(12) EUROPEAN PATENT APPLICATION

(43) Date of publication:
24.04.1996 Bulletin 1996/17

(51) Int Cl.$^6$: A61K 7/42

(21) Application number: 95850083.7

(22) Date of filing: 28.04.1995

(84) Designated Contracting States:
DE FR GB

(30) Priority: 20.10.1994 US 327517

(71) Applicant: CALIFORNIA SUNCARE INC.
Los Angeles, California 90024 (US)

(72) Inventors:
• Burmeister, Frederick H.
Little silver, NJ 07739 (US)
• Scholz, Durant B.
Chestnut Ridge, NY 10977 (US)

• Malstrom, Ivar W.
Matawan, NJ 07747 (US)
• Bennet, Suellen
Edison, NJ 08817 (US)
• Brooks, Geoffrey J.
West Orange, NJ 07052 (US)
• Adams, April
Los Angeles, CA 90024 (US)

(74) Representative: Roth, Ernst Adolf Michael et al
GÖTEBORGS PATENTBYRA AB
Box 5005
S-402 21 Göteborg (SE)

(54) **Liposome delivery of compositions to enhance tanning and repair UV damage**

(57)     The present invention concerns the delivery in carrier liposomes of novel combinations of two or more active ingredients designed to enhance tanning and ameliorate damage to skin and DNA caused by UV radiation.

## Comparison of Melanin Production

FIG. 1

## Description

Field of the Invention

This invention is directed to the delivery of active ingredients designed to enhance the tanning response to ultraviolet ("UV") exposure and to ameliorate the harmful effects of such exposure.

Background of the Invention

Exposure to UV light, whether from natural sunlight or from artificial sources, has both beneficial and harmful effects upon the skin and general health of exposed individuals. Among the benefits, UV exposure stimulates formation of Vitamin D in the skin, improving calcium absorption. Further, as evidenced by the rapid growth of the indoor tanning bed industry and the millions of outdoor sunbathers, many people believe that tanning enhances physical attractiveness.

The harmful effects of UV exposure range from mild to potentially life-threatening. In the short term, many have experienced the pain and blistering of "sunburn" resulting from UV over-exposure. Of graver concern, a strong association exists between long term over-exposure to UV radiation and a variety of human skin cancers, such as premalignant keratoses and a variety of malignant basal cell epitheliomas and squamous cell epitheliomas. These cancers may be caused, in part, by free radicals produced by UV-induced oxidation reactions.

In addition to skin cancers, over-exposure to UV radiation can lead to premature aging of the skin, particularly in light-eyed, fair-skinned individuals. The external characteristics of this condition include dry, wrinkled, thin skin with diminished elasticity. This loss in elasticity is associated at the cell and tissue level with decreases in collagen, a gelatinous protein which gives strength and flexibility to connective tissues, and hydroxyproline, a proline-derived amino acid present in collagen.

At the DNA level, UV exposure induces formation of thymine dimers which block both DNA transcription and replication, and which may be associated with the development of certain skin cancers.

Melanin, a dark, UV absorbing pigment produced by melanocytes, is one of the body's primary natural defenses against harmful UV exposure. Melanin is a polymer formed by oxidation of the amino acid tyrosine. The physiological response known as "tanning" is actually the result of UV-induced melanin formation.

The conventional approach to mitigating the effects of harmful UV radiation has been primarily directed to "sunscreen" products, which contain active ingredients that absorb or reflect radiation in the UV-A and UV-B wavelength ranges. These active ingredients, called UV filters, have been specifically identified and listed in a series of FDA regulations which establish conditions for the safety, effectiveness, and labeling of over-the-counter sunscreen products.

Conventional sunscreens may provide some protection from the harmful effects of UV over-exposure by reducing the amount of UV radiation which penetrates the outer epidermis, called the stratum corneum, to reach the underlying layers of living dermis. However, the UV filters present in sunscreens disadvantageously increase the amount of UV exposure time required to achieve a tan of a given intensity.

No product in the existing art successfully resolves the dual objectives of contemporary sunbathers who desire the aesthetic benefits of a rich tan, but, well aware of the adverse effects of UV exposure, also wish to minimize the consequent damage to their skin and general health. While the art has advanced in some respects, existing approaches tend to fall into one of two camps, one which emphasizes enhanced tanning, and another primarily aimed at protecting the skin and DNA from UV damage. For example, Meybeck et al.'s U.S. Patent No. 5,290,562, incorporated herein by reference, describes liposomal delivery of tyrosine and tyrosine derivatives for enhanced melanin formation in the hair and skin. Meybeck et al. does not, however, suggest a way to ameliorate and/or repair cellular, tissue and DNA damage caused by UV exposure.

Alternatively, liposomal delivery of a DNA repair enzyme, phage T4 endonuclease V ("T4NV"), has been reported to reverse UV-induced DNA damage in cultured human cells and to protect against photo-carcinogenesis in animal models. Ceccoli et al., J. *Invest. Dermatol.,* 93:190-194 (1989). The T4NV enzyme has also been reported to enhance UV-induced melanogenesis. Gilchrest et al., J. *Invest. Dermatol.,* 101:666-672 (1993). Further, references reporting the use of DNA repair enzymes in carrier liposomes are directed to repair of DNA level damage, and do not suggest ways to repair UV damage at the cell and tissue level.

In any case, the use of liposomes to encapsulate and deliver UV-related active ingredients represents a distinct advance over the simple emulsions of earlier tanning products. Liposomes may be thought of as "empty cells" in which the contents can be programmed. They are small, hollow spheres which comprise one or more cell membrane-like phospholipid bi-layers, enclosing one or more aqueous internal compartments. Phospholipids generally comprise fatty acids, glycerol or dyglycerides, and phosphoric acid linked to an organic base such as, for example, choline. Lecithin is a natural phospholipid found in all living organisms which has become a key building block for liposomes used in the cosmetics industry.

In contrast to the simple emulsions of earlier sunscreen lotions, liposome-based tanning products have been shown to be resistant to unwanted removal from the skin. Moreover, the similarity in the compositions of liposome membranes and animal cell membranes gives liposomes high keratin substantivity, so that encapsulated active ingredients are readily transported past the stratum corneum into the deep layers of living dermis where melanocytes exist and melanin formation occurs.

Nonetheless, the hydrophobicity or aqueous instability of certain classes of useful compounds have limited the utility of carrier liposomes. In particular, certain vitamins, such as Vitamins A and E, known to revitalize sun-damaged skin and exert anti-photocarcinogenic effects, have an affinity for oily substances. Similarly, Vitamin C is highly unstable in water. As a result, these compounds have generally been thought unsuitable for encapsulation with hydrophilic active ingredients, such as tyrosine, tyrosine derivatives and DNA repair enzymes.

Furthermore, liposome formation and active ingredient encapsulation are both highly inefficient processes. Typically, as little of 30% of the active ingredients are encapsulated in liposomes. Likewise, only a fraction of the phospholipids are incorporated in liposome membranes. Heretofore, there has been no way to adequately utilize these residual phospholipids and unencapsulated active ingredients, resulting in considerable waste.

Summary of the Invention

The present invention is directed to the liposomal delivery of novel combinations of two or more active ingredients into the living dermis. In a preferred embodiment, delivery is by a surfactant delivery system in which carrier liposomes are emulsified in one or more high hlb surfactants. "Hlb" refers to the hydrophile/lipophile balance of a surfactant molecule. Specifically, it is the numeric ratio of hydrophilic to lipophilic structures. Therefore, a "high hlb" surfactant is one that is strongly hydrophilic in nature and is primarily utilized to solubilize or emulsify lipidic materials into aqueous systems.

The value of the combined delivery system of the preferred embodiment lies in the relative inefficiency of liposome formation. In the present invention, the surface tension reducing properties of high hlb surfactants are utilized to enhance delivery of unencapsulated active ingredients into the dermis.

In its most basic conception, the present invention comprises a liposome encapsulating a first active ingredient comprising tyrosine and/or one or more tyrosine derivatives, and at least a second active ingredient, selected from the group consisting of one or more vitamin/polypeptide complexes, one or more metal ions, one or more metal/glycoprotein complexes, one or more DNA repair enzymes, and forskolin.

Generally, the second of the at least two active ingredients ameliorates UV-related damage to DNA or skin, and may also promote enhanced melanin formation. In a few cases, the second active ingredient further promotes melanin formation, but does not repair UV damage.

A preferred embodiment further comprises one or more additional members of the group from which the second active ingredient is selected, namely, one or more of those group members not selected as the second active ingredient.

The one or more vitamin/polypeptide complexes of the invention moisturize and revitalize UV damaged skin. The vitamin/polypeptide complexes of some preferred embodiments are also thought to exert anti-photocarcinogenic effects due to anti-oxidant and/or free radical scavenging properties. Other preferred vitamin/polypeptide complexes are believed to play a role in the synthesis of collagen, a protein known to be destroyed by long term UV over-exposure with a resulting loss in skin elasticity. In pure form, the vitamins of the invention are unsuitable for delivery in an aqueous carrier due to instability in aqueous solutions or hydrophobicity. However, when complexed with one or more suitable polypeptides, as more fully described below, the conjugated vitamins become water dispersible. In addition to enhancing water dispersability, these polypeptides also improve keratin substantivity and reduce toxicity.

In a more preferred embodiment, the one or more vitamin/polypeptide complexes of the invention comprise one or more of a Vitamin A/polypeptide complex, a Vitamin C/polypeptide complex and a Vitamin E/polypeptide complex. In the most preferred embodiment, each of the three above-named vitamin/polypeptide complexes is present.

In a preferred embodiment, the polypeptide of the Vitamin C/polypeptide complex comprises one or more proteins which naturally occur in Vitamin C plant sources. Preferably, the Vitamin C/polypeptide complex is further conjugated with one or more bioflavinoids.

Preferably, the Vitamin A of the Vitamin A/polypeptide complex is in the form of retinyl palmitate, produced by esterifying Vitamin A with palmitic acid, or in the form of retinol, the alcohol form of Vitamin A. When conjugated with a suitable polypeptide, the retinyl palmitate or retinol of the preferred embodiments become water dispersible. Preferably, the retinyl palmitate or retinol is complexed with one or more carrot proteins. More preferably, the retinyl palmitate/carrot protein or retinol/carrot protein complex is further conjugated with one or more beta carotenoids.

In another preferred embodiment, the Vitamin E (also called tocopherol) of the Vitamin E/polypeptide complex is esterified with nicotinic acid to produce tocopherol nicotinate, and then conjugated with a protein. Preferably, the protein of the Vitamin E/polypeptide complex, is a carotenoid. The Vitamin E/protein complex is, in addition to antioxidant/free radical scavenger, a vasodilator and antiinflammatory agent.

3

In another preferred embodiment, the one or more metal ions of the present invention include magnesium, copper, and zinc. Each of these metal ions is believed to promote melanin formation. More specifically, magnesium (Mg) is believed to stimulate formation of adenosine 3',5'-monophosphate ("cyclic AMP" or "cAMP") which promotes tyrosinase activity. Tyrosinase (also called tyrosine hydroxylase) catalyzes the oxidation of tyrosine to dihydroxyphenylalanine (L-DOPA), which is converted to melanin. Copper (Cu) is a tyrosinase co-factor which positively regulates tyrosinase activity. Zinc (Zn) functions as a co-factor to increase Cu activity, and serves as biocatalyst for many enzymatic processes in the skin. In addition to promoting melanin formation, the foregoing metal ions are also believed to remediate UV damage. Zinc, in particular, is a structural component of super-oxide dismutase, a key cellular anti-oxidant. J.J.R. Prausto DaSilva and R.J.P. Williams, The Biological Chemistry of the Elements, Oxford University Press (1991). Zinc also plays a role in maintaining the collagen matrix, perhaps through zinc-finger regulation of enzymes critical to collagen cross-linking.

In another preferred embodiment, the one or more metal/glycoprotein complexes of the invention include a Mg/glycoprotein complex, a Cu/glycoprotein complex, and a Zn/glycoprotein complex. In a more preferred embodiment, the glycoproteins are yeast glycoproteins. The glycoproteins of the metal/glycoprotein complexes enhance keratin substantivity, detoxify the metal ions and enhance moisture-binding properties.

The forskolin of the invention is extracted from the plant Coelus forskohlii. Forskolin activates adenylate cyclase, the enzyme that catalyzes the conversion of ATP to cyclic AMP, which, in turn, stimulates tyrosinase activity. In addition to catalyzing CAMP formation, forskolin is a vasodilator which improves microcirculation and reduces UV-associated inflammation. Preferably, the forskolin of the invention is a water soluble derivative of forskolin. Several such water soluble derivatives have been synthesized, including, among other forskolin derivatives suitable for use in the present invention, 7β desacetyl-7β[γ-(N-methylpiperazino)-butyryl] forskolin.

The one or more DNA repair enzymes of the present invention are of the type which can repair UV-damaged DNA. Preferably, the DNA repair enzyme of the invention is a photolyase. More preferably, said enzyme is present in a cell extract from *Micrococcus leuteus*.

In another preferred embodiment, an active ingredient, in addition to the at least two active ingredients described above, is cyclic AMP. Cyclic AMP is known to activate certain protein kinases, including tyrosinase.

Another more preferred embodiment will comprise, in addition to the liposome or cryptoliposome/surfactant delivery system and the at least two active ingredients, certain additional ingredients, comprising a nucleotide fraction, preferably yeast-derived, to enhance substantivity and support DNA replication and transcription activity; and/or one or more sequestering agents; and/or one or more preservatives.

Another potential application of the present invention is in restoring pigment to UV-damaged, treatment-damaged, or aging hair. Hair melanin is produced by biochemical events similar to those responsible for melanin production in the dermis. The melanocytes in human hair reside in the hair bulb. Studies of mouse hair follicular melanocytes suggest that hair color is related to melanogenic activity. Burchall S.A., et al., J. Endocr. 111:233-237 (1986). It is widely recognized that human brown and red hair lightens upon exposure to intense solar radiation. The darkening of light-colored hair into middle age has also been widely observed, as has the progressive age-associated depigmentation of human hair, beginning in the fourth and fifth decades of life. One method of the invention thus comprises topical application of an effective amount of the compositions of the invention to hair undergoing UV-, treatment-, or age-associated loss of pigmentation, in order to stimulate melanogenesis activity and restore lost pigmentation.

In another aspect, the present invention comprises a method for topical application of an effective amount of the compositions of the invention to human skin, either before, during or after UV exposure, to stimulate melanogenesis and/or rejuvenate and repair UV-damaged skin.

The compositions of the invention are suitable for addition to many commercially known cosmetic vehicles (or "bases"), including, without limitation, gels, lotions, creams and sprays. Typically, the concentration of the compositions of the invention in a suitable cosmetic base will be from about 0.1% to about 5.0% of the total by weight. More preferably, the concentration will be from about 1.0% to about 2.0%.

Brief Description of the Drawings

Fig. 1 is a graph showing the percentage melanin increase in four groups of test mice versus a control group.

Fig. 2 is a graph showing the percentage melanin increase in four groups of test mice versus a baseline established by melanin concentration in the control group.

Detailed Description of the Invention

The present invention concerns the liposomal delivery of a novel combination of at least two active ingredients into the dermis. The liposomes of the invention are primarily phospholipids and water. Preferably, the water is demineralized water. Preferably, the phospholipids of the invention are primarily phospholipid choline esters of phosphoric acid and a mixture of fatty acid digylcerides. Without limiting the present invention, lecithin, found in all living organisms, is a naturally occurring phospholipid suitable for use in the present invention. Plant-derived lecithin contains high levels of phosphatidylcholine and has excellent color and odor stability for use in cosmetic products. Preferably, soybeans provide an especially rich source of lecithin. Phosphatidyl compounds suitable for use in the present invention can be commercially obtained from the Sigma Chemical Company. Preferably, the phospholipids of the invention are purified to reduce or eliminate unwanted compounds, including pesticides and heavy metals. The purified phosphlipids should have a phosphatidyl choline content of greater than about 25%.

Phospholipids are present in the compositions of the invention in a concentration ranging from about 1 to about 2 percent by weight. More preferably, the concentration range is from 1.2 to about 1.6%. Methods suitable for making the liposomes of the invention were disclosed in Redziniak et al., U.S. Patent No. 4,621,023; Oleniacz, U.S. Patent No. 3,957,971; and Redziniak et al., U.S. Patent No. 4,508,703, all of which are incorporated herein by reference.

In a preferred embodiment, the delivery system of the invention is a cryptoliposomal/surfactant delivery system in which the liposomes of the invention are emulsified in one or more high hlb surfactants. A suitable hlb range for the high hlb surfactants of the invention ranges from about 10 to about 14. Preferably, the hlb range is from about 12 to about 14. Among the high hlb surfactants suitable for the present invention are ethoxylated fatty alcohols, ethoxylated sorbitan esters, and ethoxylated phosphate esters and their salts. Preferably, high hlb surfactants suitable for use in the invention include polysorbate 20, polysorbate 60, polysorbate 80, ethoxylated oleyl alcohol and diethylolamine salts of phosphated ethoxylated oleyl alcohol. More preferably, the surfactants comprise polysorbates 20, 60 and 80, also called polyoxyethylene (20, 60, or 80) sorbitan monolaurate. Most preferably, the surfactant of the present invention is polysorbate 20. However, those skilled in the art will recognize that any number of high hlb surfactants, now known or to be discovered, are suitable for the practice of the present invention. The polysorbate compounds, the ethoxylated oleyl alcohol and the diethylolamine salts of phosphated ethoxylated oleyl alcohol can be commercially obtained from the Simga Chemical Company or from Brooks Industries, Inc. The ratio of phospholipid to high hlb surfactant in the cryptoliposome/surfactant delivery system of the preferred embodiment is about 1.0 to 0.1. Preferably, that ratio is about 1.0 to 0.5.

Without limiting the invention, a suitable method for making a cryptoliposomal/surfactant delivery system suitable for the practice of the invention is the following:

1. Batch water is charged to a jacketed stainless steel vessel equipped with a high shear, homogenizing agitator and heated to from about 40 to about 45 degrees Celsius.
2. Purified phospholipids are added and the batch is mixed until fully dispersed.
3. A suitable high hlb surfactant is added and the batch is mixed until uniform.
4. The batch is passed through a microfluidizer until a particle size of about 100 to about 200 microns is achieved.
5. The pH is adjusted to from about 6.0 to about 6.5 with aminomethylpropanol.

The first of the at least two active ingredients of the present invention comprises tyrosine and/or one or more tyrosine derivatives. Preferably, the tyrosine derivatives of the invention include those disclosed in Tur, U.S. Patent No. 4,844,884, incorporated herein by reference. More preferably the tyrosine derivatives of the present invention comprise N-acetyl tyrosine, N-myristoyltyrosine, N-palmitoyltyrosine, and N-stearoyltyrosine. Most preferably, the tyrosine derivative is N-acetyl tyrosine. However, those skilled in the art will recognize that many tyrosine derivatives, including those now known and to be discovered, will be suitable for the practice of the present invention. Tyrosine, N-Acetyl tyrosine, and some of the other tyrosine derivatives suitable for the present invention can be commercially obtained from the Sigma Chemical Company. Other suitable tyrosine derivatives can be made by processes well known in organic chemistry. In a suitable embodiment, the tyrosine and/or one or more tyrosine derivatives are present in the compositions of the invention at a concentration ranging from about 5% to about 15% by weight. In a preferred embodiment, the concentration ranges from about 10% to about 12% by weight.

In more a preferred embodiment, some or all of the tyrosine and/or one or more tyrosine derivatives of the invention are present as a component of an amino acid fraction , which includes proteins, protein fragments and amino acids, preferably extracted from plant sources according to known methods. A wide range of plants is suitable for the practice of the invention. In a preferred embodiment, the amino acid fraction is extracted from a legume or from silk. In a more preferred embodiment, the amino acid fraction is extracted from Sunflower petals or Golden Legume (formerly Golden Pea). Suitable methods for making a plant-extracted amino acid fraction include, among others, alkaline hydrolysis and enzyme hydrolysis. Without limiting the invention, a suitable method for alkaline hydrolysis of an amino acid fraction from crude plant materials is as follows:

1. Plant material is dried and ground to a course powder using a hammer mill or other suitable device.
2. The powder is charged to a stainless steel vessel equipped with a heating/cooling jacket and a mixing apparatus.
3. Sufficient water is added to the vessel to cover the powder, and heated to about 50 degrees Celsius.

4. The pH is raised with NaOH to from about 10.0 to about 12.0 and the batch is agitated.
5. When sufficient protein has been extracted, the pH is adjusted to about 7.0 with citric acid and filtered.
6. Excess water may be removed by thin film evaporation.

A suitable method for enzyme hydrolysis of plant amino acids from crude plant materials is as follows:

1. After first reducing the pH to from about 5.0 to about 6.0, follow steps 1-5 above.
2. Add an effective amount of a suitable enzyme. Suitable enzymes include, without limitation, bromelain, papain, and alkalase. Allow the batch to react for from about 4 hours to about 24 hours.
3. Stop the enzyme reaction by heating to above 70 degrees Celsius for one hour to denature the enzyme. Filter and concentrate as before.

Following amino acid extraction, the tyrosine component of the fraction is concentrated relative to other amino acids by methods well known in the art. Among the suitable methods are paper chromatography, gel electrophoresis and molecular filtration. Tyrosine is present in the amino acid fraction of the invention at a concentration of from about 25% to about 35%. Preferably, tyrosine constitutes from about 28% to about 32% of the amino acid fraction. Most preferably, the tyrosine concentration of the amino acid fraction is about 30%. A plant-extracted amino acid fraction suitable for the practice of the invention can be commercially obtained from Brooks Industries, Inc. under the trademark Helioprotein.®

In a suitable embodiment, the second of the at least two active ingredients is a member selected from the group consisting of one or more vitamin/polypeptide complexes, one or more metal ions, one or more metal/glycoprotein complexes, one or more DNA repair enzymes, and forskolin.

A preferred embodiment further comprises a third active ingredient that is different than the second active ingredient and that is selected from the group consisting of one or more vitamin/polypeptide complexes, one or more metal ions, one or more metal/glycoprotein complexes, one or more DNA repair enzymes, forskolin, and mixtures thereof.

In a preferred embodiment, the one or more vitamin/polypeptide complexes of the invention comprise one or more of a Vitamin C/polypeptide complex, a Vitamin A/polypeptide complex, and a Vitamin E/polypeptide complex. In a more preferred embodiment, each of the three above-named vitamin/polypeptide complexes is present.

Preferably, the Vitamin C/polypeptide complex of the invention will be conjugated with water soluble proteins naturally present in Vitamin C sources. The best sources of Vitamin C are citrus fruits, Rose Hips, acerola cherries, and fresh tea leaves. More preferably, the Vitamin C complex will also be conjugated with additional such naturally-occurring compounds, including the bioflavinoids. Without limiting the present invention, a suitable Vitamin C/polypeptide complex can be obtained by placing finely minced citrus pulp, or other minced Vitamin C source, in a stainless steel fermentation vessel and reacting at from about 37° to about 40° C with an effective amount of synthetic ascorbic acid. The preferred ratio of minced pulp to synthetic ascorbic acid ranges from about 5:1 to about 3:1. More preferably, the ratio ranges from about 4.7:1.0 to about 3.7:1.0. Synthetic ascorbic acid can be obtained from the Sigma Chemical Company. The reaction should be allowed to proceed until no more free-state ascorbic acid is detectable in the reaction mixture by standard assay. The reaction normally reaches completion in about 4 to 6 hours. At this point, the reaction is stopped, the solids are removed by filtration, the remaining pulp is washed and the washings are combined with the first extract. The solution is concentrated in vacuo or under nitrogen protection, followed by spray-drying to a free-flowing white to cream powder. A Vitamin C/polypeptide complex suitable for the present invention can also be commercially obtained from Brooks Industries, Inc., marketed under the trademark Vitazyme™ C. Preferably, the Vitamin C/polypeptide complex of the invention is present in the composition of the invention at a concentration ranging from between about 0.1 % and about 5.0% by weight. More preferably the concentration range is from about 1.0% to about 1.5% by weight.

Preferably, the Vitamin A of the Vitamin A/polypeptide complex is in the form of retinol. More preferably, the Vitamin A of the complex is in the form of retinyl palmitate (or Vitamin A palmitate) or in the form of retinol. Preferably, the polypeptide of the Vitamin A/polypeptide complex is a carrot protein. Most preferably, a retinyl palmitate/carrot protein complex is further conjugated with one or more beta carotenoids. Methods for preparing a Vitamin A/polypeptide complex suitable for the present invention are disclosed in Schaeffer et al., U.S. Patent No. 5,124,313, incorporated herein by reference. Preferably, the retinyl palmitate/polypeptide complex or retinol/polypeptide complex of the invention is present in a concentration ranging from about 0.1% to about 5.0% by weight. More preferably, the concentration range is from about 1.0% to about 1.5% by weight.

In a preferred embodiment, the Vitamin E of the Vitamin E/polypeptide complex is esterified with nicotinic acid to produce tocopherol nicotinate and then conjugated with a suitable polypeptide. Preferably, the polypeptide of the Vitamin E/polypeptide complex comprises one or more carotenoids. In another preferred embodiment, the tocopherol compound to be conjugated with a suitable polypeptide is tocopherol acetate. Tocopherol, tocopherol nicotinate and tocopherol acetate can each be commercially obtained from the Sigma Chemical Company.

Without limiting the present invention, a suitable method for preparing the Vitamin E/polypeptide complex of the invention is to combine soybean, wheat germ, and corn pulp in a reaction vessel with about 0.25% tocopherol. The reaction is allowed to proceed until no free state tocopherol is detected by an HPLC assay. A tocopherol nicotinate/polypeptide complex suitable for the present invention can also be commercially obtained from Brooks Industries, Inc., marketed under the trademark Vitazyme™ E. Brooks Industries, Inc. also markets a product, Vitazyme ACTN™, which consists of equal parts of Vitazyme A™, Vitazyme C™ and Vitazyme E™, that is suitable for the practice of the invention.

The Vitamin E/polypeptide complex of the invention is present in a concentration ranging from about 0.01% to about 0.2% by weight. In a preferred embodiment, the concentration ranges from about .02% to about .07%.

In a preferred embodiment, the one or more metal ions of the present invention include one or more of magnesium, copper, and zinc. The metal ions of the present invention can be commercially obtained from the Aldrich Chemical Company. In a suitable embodiment, the magnesium ion is present in the composition of the invention at a concentration of from about 0.01% to about 0.1%. Preferably, the magnesium ion concentration is about 0.05%. In a suitable embodiment, the copper ion is present in the composition of the invention at a concentration of from about 0.0005% to about 0.01%. Preferably, the copper ion concentration is about 0.001%. In a suitable embodiment, the zinc ion is present in the composition of the invention at a concentration of from about 0.001% to about 0.01%. Preferably, the zinc ion concentration is about 0.005%.

The one or more metal/glycoprotein complexes of a more preferred embodiment comprise one or more of a Mg/glycoprotein complex, a Cu/glycoprotein complex, and a Zn/glycoprotein complex. Preferably, the glycoproteins are yeast glycoproteins. Without limiting the present invention, a Mg/glycoprotein complex suitable for the present invention can be manufactured according to the following method:

1. A suspension of live yeast cells in an appropriate culture medium is prepared in a glass-lined reaction vessel and brought to full viability. Magnesium ions are introduced into the suspension where they are taken up by the yeast cells and complexed with yeast glycoproteins. The reaction is monitored by means of an HPLC assay procedure until no free inorganic magnesium is present in the mixture.

2. The remaining live yeast is autolyzed using a proteolytic enzyme.

3. The cell walls are removed by filtration and the liquid is concentrated and spray-dried to a free-flowing, cream colored solid.

A Mg/glycoprotein complex suitable for the present invention is also commercially available from Brooks Industries, Inc. under the trademark Acqua-Biomin® Mg/P/C.

Without limiting the present invention, a Cu/glycoprotein complex suitable for use in the invention can be made according to the methods recited for manufacture of the Mg/glycoprotein complex with the difference that copper ions are introduced into the yeast suspension in place of magnesium ions. The solid produced by the Cu/glycoprotein complex will be a white to greenish solid. A Cu/glycoprotein complex suitable for the present invention is also commercially available from Brooks Industries, Inc. under the trade name Acqua-Biomin® Cu/P/C.

Without limiting the present invention, a Zn/glycoprotein complex suitable for use in the invention can be made according to the method used for the Mg and Cu/glycoprotein complexes, with the difference that zinc ions are introduced into the yeast suspension rather than magnesium or copper ions. The solid produced by the Zn/glycoprotein complex will be white to cream in color. The Mg/glycoprotein complex is present in the composition of the invention at a concentration ranging from about 1.5% to about 2.5%. Preferably, the concentration is about 2.0%. The Zn/glycoprotein complex is present at a concentration ranging from about 0.5% to about 1.5%. Preferably, the concentration is about 1.0%. The Cu/glycoprotein is present at a concentration of about 0.5% to about 1.5%. Preferably, the concentration is about 1.0%

The forskolin of the present invention is an extract from the plant Coelus forskohlii. Coelus forskohlii extract can be commercially obtained from the Sigma Chemical Corporation. In a preferred embodiment, the forskolin of the invention is a water soluble derivative of forskolin. In another preferred embodiment, the forskolin is the forskolin derivative 7β desacetyl-7β[γ-(N-methylpiperazino)-butyryl] forskolin. However, those skilled in the art will recognize that many water soluble forskolin derivatives, including those now known, or to be discovered, will be suitable for the practice of the invention. Forskolin should be present in a concentration ranging from about .0001% to about 0.003%. Preferably, the concentration is about .001%.

The one or more DNA repair enzymes of the present invention will be of the type which can repair UV-damaged DNA. Examples of such enzymes include the photolyases, the UV-DNA endonucleases, the UV-DNA helicases, the UV-DNA base excision repair gylcosylases, the apurinic/apyrimidinic endonucleases, and similar enzymes now known or subsequently identified. Suitable DNA repair enzymes can be obtained from a variety of sources, including bacterial sources. The DNA repair enzyme of the present invention may be present in cell extracts or in pure form. Methods for purifying DNA repair enzymes and administering such enzymes in liposomes are disclosed in Yarosh, U.S. Patent No. 5,272,079, incorporated herein by reference. Preferably, for the practice of the present invention, cell extracts are used.

More preferably, photolyase-containing extracts from *Micrococcus* leuteus (formerly *Anacystis nidulans)* are used. DNA repair enzymes and enzyme-containing extracts suitable for use in the present invention can be commercially obtained from Applied Genetics, Inc. Without limiting the present invention, liposomal encapsulation of DNA repair enzymes has been described in Ceccoli et al., J. *Invest. Dermatol.,* 93:190-194 (1989), incorporated herein by reference.

In another preferred embodiment of the present invention, an active ingredient, in addition to the at least two active ingredients described above, is cyclic AMP. Cyclic AMP is a tyrosine activator which can be commercially obtained from the Aldrich Chemical Company. Cyclic AMP is present in a concentration ranging from about .004% to about 0.1. More preferably, the concentration ranges from 0.01% to about 0.1% by weight.

Another preferred embodiment of the present invention will comprise, in addition to the liposome or cryptoliposome/surfactant delivery system and the at least two active ingredients, certain additional ingredients. One such preferred embodiment will include a nucleotide fraction. Preferably, the nucleotide fraction of the invention is extracted from yeast cultures. Yeast-derived nucleotide fractions suitable for use in the preferred embodiment can be obtained from Brooks Industries, Inc. under the trade name Bioplex™ RNA. Preferably, the nucleotide fractions are present in the compositions of the invention at a concentration ranging from about 0.1% to about 5.0% by weight. More preferably, the range is from about 0.5% to about 1.0% by weight.

Another such preferred embodiment further comprises one or more of sequestering agents, preservative agents, and buffers. The sequestering agents are of the type that may react with and entrap metal ions from aqueous solution. Suitable sequestering agents include, without limitation, sodium salts of ethylenediaminetetraacetic acid, hydroxyethlethylenediaminetetraacetic acid, nitrilotriacetic acid, and diethylenetriaminepentaacetic acid, and combinations thereof. Tetrasodium EDTA is a suitable sequestering agent which can be commercially obtained from ICN Biomedicals, Inc. Suitable preservatives include, without limitation, esters of p-hydroxybenzoic acid, admantane derivatives, hydantoin derivatives, and combinations thereof. Methyl paraben, dimethyl dimethylolhydantoin, and Dowicil™ 200 are suitable preservatives. Methyl paraben can be obtained from Sigma Chemical Company. Dowicil™ 200 can be commercially obtained from Dow Chemical. Buffers suitable for use in the invention include, without limitation, partially or completely neutralized salts of weak multivalent acids or bases. For example, aminomethylpropanol (AMP) is a suitable buffer which can be commercially obtained from ICN Biomedicals, Inc. In that regard, a suitable pH range for the compositions of the invention is from about 4.5 to about 6.5. Preferably, the pH range is from about about 5.5 to about 6.0. In the most preferred embodiment, the pH of the composition is about 6.0.

Those skilled in the art will recognize that any one of a large number of sequestering agents, preservatives and buffers, in addition to those named above, are suitable for the practice of the invention.

In another aspect, the invention comprises a method for restoring lost pigment to human hair comprising the topical application of an effective amount of the compositions of the invention to the hair. An effective amount ranges from about 0.0001 gram to about 0.1 gram of a composition of the invention per gram of hair treated.

More preferably, the invention comprises a method to enhance tanning, revitalize the skin and repair UV damage, comprising the topical application of an effective amount of the compositions of the invention to the skin before, during or after UV irradiation. An effective amount is generally from about 0.0001 ml/square centimeter to about 0.1 ml per square centimeter of skin surface area. Preferably, the amount is about 0.02 ml per square centimeter of skin surface area. In a suitable method, the composition of the invention is applied from about 24 hours before UV exposure to about 48 hours following UV exposure. In a more preferred method, the composition of the invention is applied between about 2-3 hours before UV exposure and about 4-6 hours following such exposure. More preferably, the composition of the invention is applied from about one half hour before UV exposure to about 1 hour following exposure.

The compositions of the invention are suitable for addition to many commercially known cosmetic bases, including, without limitation, oil in water emulsions, such as lotions and creams, and also including gels, and sprays. These cosmetic bases may also contain ingredients to enhance color and fragrance. Typically, the concentration of the compositions of the invention in a suitable base will be from about 0.1% to about 5.0% of the total by weight. More preferably, the concentration will be from about 1.0% to about 2.0%.

The invention will be further clarified by consideration of the following examples, which are intended to be purely exemplary of the use of the invention.

Example 1

[FORMULATION 1]

| INGREDIENT | % by Weight | Gm |
|---|---|---|
| Helioprotein™ Plant Amino Acid Extract | 40.00 | 400.0 |
| Water | 24.90 | 249.0 |
| Bioplex™ RNA Powder | 0.50 | 5.0 |
| Methyl Paraben | 0.20 | 2.0 |
| Highly Purified Phospholipids | 10.00 | 100.0 |
| Vitazyme™ ACTN | 20.00 | 200.0 |
| Dowicil™ 200 | 0.20 | 2.0 |
| Acqua-Biomin™ Mg | 2.00 | 20.0 |
| Acqua-Biomin™ Cu | 1.00 | 10.0 |
| Acqua-Biomin™ Zn | 1.00 | 10.0 |
| AMP-95 | qs | qs |

400 gm of Helioprotein™ plant amino acid extract was added to a #316 stainless steel cylindrical vessel with an open top and equipped with a heating/cooling jacket and sanitary fittings. The extract was stirred with a variable speed Lightnin™ mixer until a vortex was formed. 249 gm water was slowly added to the vessel and stirred for 5 minutes. Five gm of Bioplex™ RNA Powder was added and stirred until dissolved. The mixture was warmed until the temperature of the mixture reached 50° C, at which time 2 gm of methyl paraben preservative was added. The temperature was not allowed to rise above 50° C. 100 gm of highly purified phospholipids was added and dispersed by stirring for one hour. The mixture was then allowed to cool. 200 gm of Vitazyme ACTN™ was added and dispersed by stirring for 5 minutes. The mixture was allowed to cool until the temperature had dropped below 40° C, after which time two gm of Dowicil 200 was added and stirred until dissolved. Twenty gm of Acqua-Biomin™ Mg was added and the mixture was stirred for 5 minutes. Ten gm of Acqua-Biomin™ Cu was added and the mixture was stirred for 5 minutes. Ten gm of Acqua-Biomin™ Zn was added and the mixture was stirred for 5 minutes. After adding the Acqua-Biomin™ series, the pH was checked and adjusted to pH 6.0 with AMP-95 (ICN Biomedicals, Inc.), as needed. The final solution was then twice passed through a Model 110-F Microfluidizer manufactured by Microfluidics Corporation.

Example 2

[FORMULATION 2]

| INGREDIENT | % by Weight | Gm |
|---|---|---|
| Helioprotein™ Plant Amino Acid Extract | 40.00 | 400 |
| Water | 25.089 | 250.89 |
| Bioplex™ RNA Powder | 0.50 | 5.0 |
| Methyl Paraben | 1.20 | 2.0 |
| Highly Purified Phospholipids | 10.00 | 100.0 |
| Vitazyme™ ACTN | 20.00 | 200.0 |
| Dowicil™ 200 | 0.20 | 2.0 |
| Acqua-Biomin™ Mg | 2.00 | 20.0 |
| Acqua-Biomin™ Cu | 1.00 | 10.0 |
| Acqua-Biomin™ Zn | 1.00 | 10.0 |
| AMP-95 | qs | qs |
| Cyclic AMP | 0.01 | 0.1 |
| Forskolin | 0.001 | 0.01 |

In addition to the ingredients used in Example 1, CAMP and forskolin were added to produce Formulation 2.

The procedures of Example 1 were followed to produce 999.89 gm of Formulation 1. After the solution had cooled to room temperature, 0.1 gm of cyclic AMP was added and stirred until dissolved. $1 \times 10^{-2}$ gm water soluble forskolin derivative was then added and stirred until dissolved. The pH of the solution was adjusted to pH 6.0 by adding AMP-95, as needed.

Example 3

[FORMULATION 3]

| INGREDIENT | % by Weight | Gm |
|---|---|---|
| Helioprotein™ Plant Amino Acid Extract | 40.00 | 400.0 |
| Water | 24.089 | 240.89 |
| Bioplex™ RNA Powder | 0.50 | 5.0 |
| Methyl Paraben | 0.20 | 2.0 |
| Highly Purified Phospholipids | 10.00 | 100.0 |
| Vitazyme™ ACTN | 20.00 | 200.0 |
| Dowicil™ 200 | 0.20 | 2.0 |
| Acqua-Biomin™ Mg | 2.00 | 20.0 |
| Acqua-Biomin™ Cu | 1.00 | 10.0 |
| Acqua-Biomin™ Zn | 1.00 | 10.0 |
| AMP-95 | qs | qs |
| Cyclic AMP | 0.01 | 0.10 |
| Forskolin | 0.001 | 0.01 |
| Ultrasome™ | 1.0 | 10.0 |

In addition to the ingredients used in Example 2, 10 gm of Ultrasome™ (Applied Genetics, Inc.) was added and then agitated using low shear agitation with a paddle type mixer. The pH was adjusted to pH 6.0 by adding AMP-95, as needed.

Example 4

[FORMULATION 4]

| INGREDIENT | % by Weight | Gm |
|---|---|---|
| Helioprotein™ Plant Amino Acid Extract | 40.00 | 400.0 |
| Water | 24.10 | 241.0 |
| Bioplex™ RNA Powder | 0.50 | 5.0 |
| Methyl Paraben | 0.20 | 2.0 |
| Highly Purified Phospholipids | 10.00 | 100.0 |
| Vitazyme™ ACTN | 20.00 | 200.0 |
| Dowicil™ 200 | 0.20 | 2.0 |
| Acqua-Biomin™ Mg | 2.00 | 20.0 |

Continuation of the Table on the next page

(continued)

| INGREDIENT | % by Weight | Gm |
|---|---|---|
| Acqua-Biomin™ Cu | 1.00 | 10.0 |
| Acqua-Biomin™ Zn | 1.00 | 10.0 |
| AMP-95 | qs | qs |
| Ultrasome™ | 1.00 | 10.0 |

In addition to the ingredients used in Example 1, 10 gm Ultrasome™ was added and then agitated using low shear agitation with a paddle type mixer. The pH was adjusted to pH 6.0 by adding AMP-95, as needed.

Example 5

A study was conducted to compare the effectiveness of Formulations 1, 2, 3, and 4 in promoting melanin formation in four groups of test mice versus a control group.

Materials and Methods

The test animal was a hairless mouse. The animals were maintained under standard laboratory conditions in a ventilated room with water and food ad.lib. Light was controlled automatically on a 12 hour on/off cycle. Five mice were selected for each product tested. The product was applied in 0.2 ml. aliquots to the backs of the animals with a finger cot. Immediately after all animals were treated, the animals were irradiated with 50 millijoules of UV-B light using four FS40 type sunlamps. Dosage was monitored with an IL Model 1700 Research Model Radiometer. Five untreated animals served as a control group. The animals were returned to the colony after irradiation, but housed in separate cages.

Seventy-two hours after irradiation the animals were sacrificed and the skin removed by dissection. A 16 cm$^2$ section was placed on a styrofoam block and placed in a water bath at 50°C for one minute. The skin was removed, immediately scraped with a scalpel, placed in 1 Normal NaOH and sonicated at 4°C to homogenize the tissue. The homogenate was mechanically mixed on a vortex mixer for 10 minutes and then centrifuged at 15,000 xg for 10 minutes at 4° C. The supernatant was removed and poured through a 1 micron glass filter. One aliquot was taken for protein analysis and a second aliquot for an optical density determination at 475 nm.

The protein was determined by the biuret method. (the Sigma Method).

Results

The optical density results* are shown in Table 1.

TABLE 1

| Test Product | Optical Density Melanin | Optical Density Protein | $\dfrac{\text{OD Melanin}}{\text{OD Protein}}$ |
|---|---|---|---|
| Control | 0.779 | 0.201 | 3.97 |
| Form. 1 | 0.952 | .189 | 5.04 |
| Form. 2 | 1.495 | .207 | 7.22 |
| Form. 3 | 1.750 | .210 | 8.33 |
| Form. 4 | 1.301 | .204 | 6.37 |

* Calculations were made as follows:

$$\% \text{ Melanin increase} = \frac{\text{OD ratio product - OD ratio control}}{\text{OD ratio control}} \times 100$$

This data shows the following rank order of Formulations 1-4 from higher melanin-inducing effectiveness to lower melanin-inducing effectiveness, as follows:

1. Formulation 3 showed a melanin increase of 109.64% over the control.
2. Formulation 2 showed a melanin increase of 81.68% over the control.
3. Formulation 4 showed a melanin increase of 60.43% over the control.
4. Formulation 1 showed a melanin increase of 26.71% over the control.

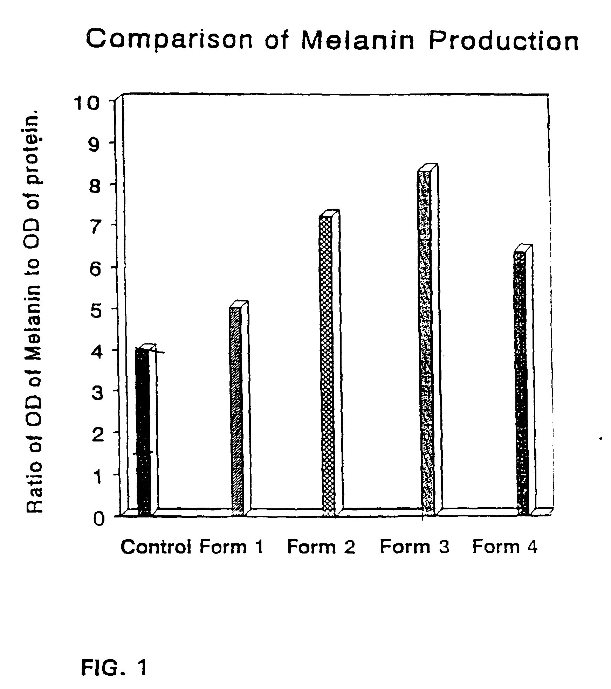

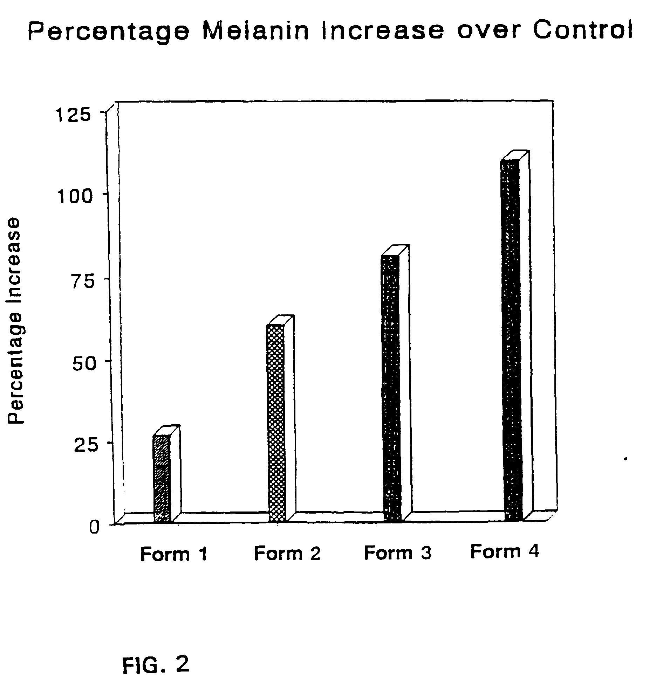

The foregoing data is represented in graphic form in Figs. 1 and 2.

Having illustrating and described the principles of the invention and several preferred embodiments, it should be apparent to those skilled in the art that the invention can be modified in arrangement and detail without departing from such principles. I claim all modifications coming within the spirit and scope of the following claims.

## Claims

1. A composition comprising:

   a first active ingredient selected from the group consisting of tyrosine, one or more tyrosine derivatives, and mixtures thereof;
   a second active ingredient selected from the group consisting of one or more vitamin/polypeptide complexes, one or more metal ions, one or more metal/glycoprotein complexes, one or more DNA repair enzymes, forskolin, and mixtures thereof; and
   a liposome encapsulating the active ingredients.

2. The composition of claim 1 further comprising cyclic AMP.

3. The composition of claim 1 wherein the liposome is emulsified in a high hlb surfactant.

4. The composition of claim 3 wherein the high hlb surfactant comprises one or more of polysorbate 20, polysorbate 60 and polysorbate 80.

5. The composition of claim 1 further comprising a third active ingredient that is different than the second active ingredient and that is selected from the group consisting of one or more vitamin/polypeptide complexes, one or more metal ions, one or more metal/glycoprotein complexes, one or more DNA repair enzymes, forskolin, and mixtures thereof.

6. The composition of claim 1 wherein the one or more vitamin/polypeptide complexes comprise one or more of a Vitamin A/polypeptide complex, a Vitamin C/polypeptide complex and a Vitamin E/polypeptide complex

7. The composition of claim 6 wherein the Vitamin A of the Vitamin A/polypeptide complex comprises Vitamin A palmitate.

8. The composition of claim 7 wherein the polypeptide of the Vitamin A palmitate/polypeptide complex comprises one or more carrot proteins.

9. The composition of claim 8 wherein the Vitamin A palmitate/carrot protein complex further comprises one or more beta carotenoids.

10. The composition of claim 6 wherein the Vitamin E of the Vitamin E/polypeptide complex comprises tocopherol nicotinate.

11. The composition of claim 10 wherein the polypeptide of the Vitamin E/polypeptide complex comprises one or more carotenoids.

12. The composition of claim 6 wherein the polypeptide of the Vitamin C/polypeptide complex comprises one or more proteins which naturally occur in Vitamin C plant sources.

13. The composition of claim 12 wherein the Vitamin C/polypeptide complex further comprises one or more bioflavinoids.

**14.** The composition of claim 1 wherein the vitamin/polypeptide complex is water dispersible.

**15.** The composition of claim 1 wherein the one or more metal ions comprise one or more of magnesium, copper, and zinc.

**16.** The composition of claim 1 wherein the one or more metal/glycoprotein complexes comprise one or more of a Mg/glycoprotein complex, a Cu/glycoprotein complex and a Zn/glycoprotein complex.

**17.** The composition of claim 16 wherein the glycoproteins comprise yeast glycoproteins.

**18.** The composition of claim 1 wherein the one or more DNA repair enzymes comprise a photolyase.

**19.** The composition of claim 18 wherein the photolyase is present in a *Micrococcus leuteus* cell extract.

**20.** The composition of claim 1 wherein the forskolin is a water soluble forskolin derivative.

**21.** The composition of claim 20 wherein the forskolin is 7β desacetyl-7β[γ-(N-methylpiperazino)-butyryl.

**22.** The composition of claim 1 wherein the tyrosine derivative comprises N-Acetyl tyrosine N-acetyl tyrosine, N-myristoyltyrosine, N-palmitoyltyrosine, and N-stearoyltyrosine.

**23.** A composition comprising:

a first active ingredient selected from the group consisting of tyrosine, one or more tyrosine derivatives, and mixtures thereof;
a second active ingredient selected from the group consisting of a Vitamin A palmitate/carrot protein complex, a Vitamin C/protein complex, a Vitamin E/ carotenoid complex, and mixtures thereof;
a third active ingredient selected from the group consisting of a Mg/glycoprotein complex, a Cu/glycoprotein complex, a Zn/glycoprotein complex, and mixtures thereof;
a fourth active ingredient comprising a photolyase;
a fifth active ingredient comprising a water soluble forskolin derivative; and
a liposome encapsulating the active ingredients.

**24.** The composition of claim 23 further comprising cyclic AMP.

**25.** A method comprising:

a first step of producing a cosmetic tanning product by adding to a suitable cosmetic base a composition comprising:
a first active ingredient selected from the group consisting of tyrosine, one or more tyrosine derivatives, and mixtures thereof;
a second active ingredient selected from the group consisting of one or more vitamin/polypeptide complexes, one or more metal ions, one or more metal/glycoprotein complexes, one or more DNA repair enzymes, forskolin, and mixtures thereof;
a liposome encapsulating the active ingredients; and
a second step of applying an effective amount of the cosmetic tanning product to the skin before, during or after exposure UV radiation.

**26.** A method comprising:

a first step of producing a hair care product by adding to a suitable cosmetic base a composition comprising:
a first active ingredient selected from the group consisting of tyrosine, one or more tyrosine derivatives, and mixtures thereof;
a second active ingredient selected from the group consisting of one or more vitamin/polypeptide complexes, one or more metal ions, one or more metal/glycoprotein complexes, one or more DNA repair enzymes, forskolin, and mixtures thereof;
a liposome encapsulating the active ingredients; and
a second step of applying an effective amount of the hair care product to the hair.

Comparison of Melanin Production

FIG. 1

Percentage Melanin Increase over Control

FIG. 2